**Europäisches Patentamt**

(19) **European Patent Office**

**Office européen des brevets**

(11) Numéro de publication: **0 095 990 B1**

(12) **FASCICULE DE BREVET EUROPEEN**

(45) Date de publication du fascicule du brevet: **18.12.85**

(21) Numéro de dépôt: **83420089.1**

(22) Date de dépôt: **31.05.83**

(51) Int. Cl.⁴: **A 61 B 17/58**

(54) Perfectionnements aux clous pour l'osthéosynthèse des fractures de cols fémoraux.

(30) Priorité: **02.06.82 FR 8209838**

(43) Date de publication de la demande:
**07.12.83 Bulletin 83/49**

(45) Mention de la délivrance du brevet:
**18.12.85 Bulletin 85/51**

(84) Etats contractants désignés:
**DE GB IT**

(56) Documents cités:
**EP - A - 0 029 752**
**CH - A - 576 249**
**FR - A - 1 037 169**

(73) Titulaire: **Société Anonyme TORNIER, chemin Doyen Gosse, F-38330 Saint Ismier (FR)**

(72) Inventeur: **Tornier, Alain, Le Brocey, F-38190 Crolles (FR)**

(74) Mandataire: **Karmin, Roger et al, Cabinet MONNIER 150, cours Lafayette, F-69003 Lyon (FR)**

ACTORUM AG

## Description

La présente invention est relative à des perfectionnements apportés aux prothèses, telles que les clous, destinées à l'ostéosynthèse des fractures de cols fémoraux.

Suivant un mode particulier d'ostéosynthèse des fractures en question, on engage des tiges préarquées de faible diamètre dans le canal médullaire du fémur afin que leur partie extrême vienne se placer au niveau de la fracture. De telles tiges sont décrites par exemple dans le brevet CH-A N° 576249.

Bien entendu, du fait de la relative souplesse des clous en question, on en utilise généralement trois de manière à constituer une armature résistante.

Le principal inconvénient des clous considérés réside dans le fait qu'ils ont une fâcheuse tendance à ressortir par leur trou d'entrée, de telle sorte que bien entendu la partie fracturée du fémur n'est plus convenablement maintenue.

Les perfectionnements qui font l'objet de la présente invention visent à remédier à cet inconvénient.

A cet effet, l'extrémité de chaque clou opposée à celle qui est destinée à venir se placer au niveau de la fracture est pourvue d'un double cambrage dont l'épaulement constitue une butée destinée à venir prendre appui dans le trou osseux.

Pour améliorer la retenue de ce clou, il se termine par une palette qui peut éventuellement être assujettie à l'os au moyen d'une vis.

Le dessin annexé, donné à titre d'exemple, permettra de mieux comprendre l'invention, les caractéristiques qu'elle présente et les avantages qu'elle est susceptible de procurer:

La fig. 1 illustre la manière dont un clou suivant l'invention est disposé à l'intérieur du canal médullaire d'un fémur.

La fig. 2 est une vue en perspective à grande échelle de l'extrémité cambrée d'un clou suivant l'invention.

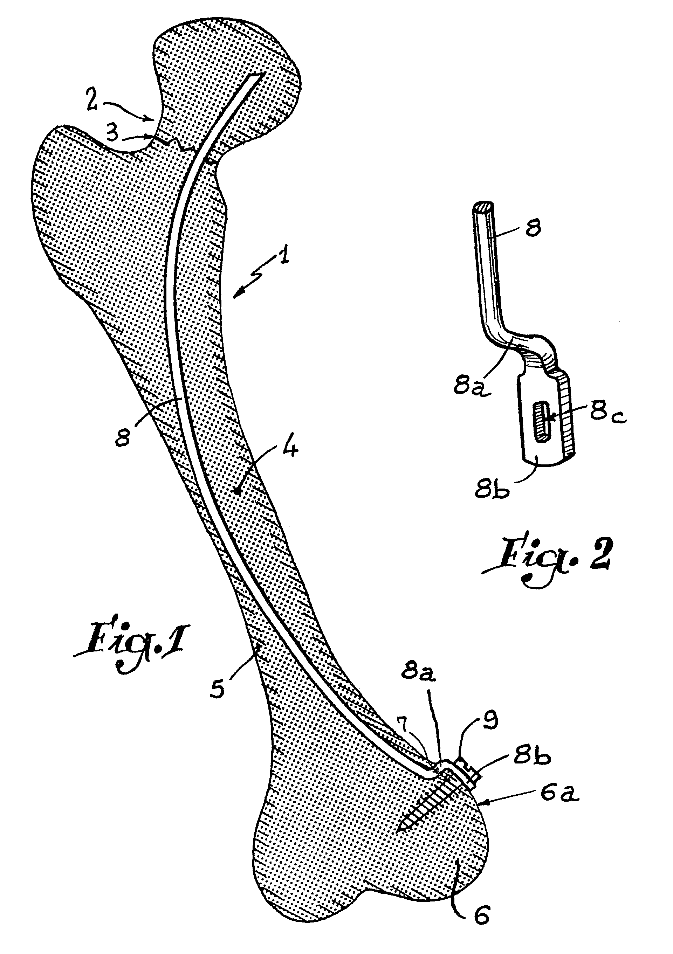

On a illustré en fig. 1 un fémur 1 dont le col 2 est fracturé suivant une ligne brisée 3. Le canal médullaire du fémur 1 a été affecté de la référence 4. L'os en question ne comprend presque plus de spongieux et seulement une corticale 5 de faible épaisseur car on a affaire à un patient âgé.

Après réduction de la fracture, on perce dans l'apophyse inférieure 6 du fémur 1 un trou 7 partant de la face interne 6a de ladite apophyse et qui débouche dans le canal médullaire 4. Après cette opération préalable un ou plusieurs clous 8 sont engagés dans le canal médullaire considéré comme cela est bien connu dans la pratique.

Mais suivant l'invention l'extrémité du clou 8 qui coopère avec le trou 7 est pourvue, comme illustré en fig. 2, d'un double cambrage, c'est-à-dire que l'extrémité en question est d'abord renvoyée pour former un épaulement 8a orienté en gros perpendiculairement à la direction générale du clou 8 puis à nouveau pliée pour réaliser une palette 8b qui, à l'état libre, est orientée parallèlement à la direction générale du clou 8. La palette 8b est pourvue d'une perforation 8c.

Bien entendu lors de l'introduction d'un clou 8 dans le canal médullaire d'un fémur 1, il subit des déformations élastiques du fait que sa forme à l'état libre ne correspond pas exactement à celle du canal médullaire en question. Dans ces conditions et grâce à sa matière constitutive, chaque clou subit une déformation notable surtout au niveau du col 2 et de l'apophyse inférieure 6. C'est évidemment grâce à ces déformations élastiques que les deux parties du col 2 sont maintenues en position après réduction de la fracture. Du fait de la déformation du clou 8 au niveau du trou 7, l'épaulement 8a qui vient se loger dans ledit trou assure une retenue efficace du clou par rapport à la corticale 5. La palette 8b qui reste à l'extérieur du trou 6 pourrait dans des cas particuliers être assujettie à la face interne 6a de l'apophyse 6 au moyen d'une vis 9 qui ne subirait pas d'efforts de cisaillement.

On a ainsi réalisé grâce à l'invention un clou pour l'ostéosynthèse des fractures de cols fémoraux qui est assuré de rester en place tant que cela est nécessaire à la recalcification même sans fixation de la palette par une vis.

## Revendications

1. Clou pour l'ostéosynthèse des fractures de cols fémoraux du genre réalisé au moyen d'une tige préarquée destinée à être engagée dans le canal médullaire (4) du fémur (1) à partir d'un trou (7) ménagé dans la face intérieure de son apophyse (6) qui constitue une partie du genou, caractérisé en ce que l'une de ses extrémités comporte un double cambrage (8a, 8b) dont l'épaulement (8a) est destiné à venir se loger dans le trou osseux (7).

2. Clou suivant la revendication 1, caractérisé en ce que son extrémité à double cambrage se termine par une palette (8b) destinée à venir reposer contre la partie externe de l'apophyse (6).

3. Clou suivant la revendication 2, caractérisé en ce que la palette (8b) est percée d'une perforation (8c) afin qu'elle puisse être assujettie à l'apophyse (6) au moyen d'une vis (9).

## Patentansprüche

1. Nagel für die Osteosynthese bei Oberschenkelhalsfrakturen mit einem Schaft mit vorgefertigter Krümmung, bestimmt zum Einführen in die Markröhre (4) des Oberschenkelknochens (1) durch eine Öffnung (7) in der Innenfläche seines Knochenfortsatzes (6), der einen Teil des Knies bildet, dadurch gekennzeichnet, dass eines der Nagelenden eine doppelte Kröpfung (8a, 8b) aufweist, deren Schulter (8a) dazu bestimmt ist, in die Knochenöffnung (7) zu liegen zu kommen.

2. Nagel nach Anspruch 1, dadurch gekennzeichnet, dass sein doppelt gekröpftes Ende in einer Palette (8b) endet, die dazu bestimmt ist, an dem äusseren Teil des Knochenfortsatzes (6) anzuliegen.

3. Nagel nach Anspruch 2, dadurch gekennzeichnet, dass die Palette (8b) ein Loch (8c) aufweist, so dass sie mittels einer Schraube (9) am Knochenfortsatz (6) befestigbar ist.

**Claims**

1. Nail for the osteosynthesis of fractures of the femur neck, of the type produced by means of a precambered rod designed to be inserted in the medullary canal (4) of the femur (1) through a hole (7) made in the inner face of its apophysis (6) which forms a part of the knee, characterised in that one of its ends incorporates a double bend (8a, 8b), the shoulder (8a) of which is designed to become lodged in the hole (7) in the bone.

2. Nail according to Claim 1, characterised in that its end having a double bend terminates in a blade (8b) designed to rest against the outer part of the apophysis (6).

3. Nail according to Claim 2, characterised in that the blade (8b) is pierced with a hole (8c) so that it may be secured to the apophysis (6) by means of a screw (9).

*Fig.1*

*Fig. 2*